# FASCICULE DE BREVET EUROPEEN

(11) **EP 0 904 054 B1**
(45) Date de publication et mention de la délivrance du brevet: **13.11.2002**
(21) Numéro de dépôt: 97925132.9
(22) Date de dépôt: 23.05.1997
(51) Int. Cl.: A61K 7/50, A23L 3/3562, A01N 31/06

(54) **UTILISATION D'alpha-ALKYLGLUCOSIDES ET D'ESTERS D'alpha-ALKYLGLUCOSIDES EN TANT QU'AGENTS EMULSIONNANTS ANTI-MICROBIENS**
VERWENDUNG VON alpha-ALKYLGLUCOSIDEN UND DEREN ESTERN ALS ANTIMIKROBIELLE EMULGATOREN
USE OF alpha-ALKYLGLUCOSIDES AND alpha-ALKYLGLUCOSIDE ESTERS AS ANTI-MICROBIAL EMULSIFYING AGENTS

(30) Priorité: 24.05.1996 FR 9606517
(43) Date de publication de la demande: 31.03.1999
(73) Titulaire: ULICE, F-63200 Riom (FR)
(72) Inventeur: BOURES, Emmanuel, F-63000 Clermont-Ferrand (FR); MESSAGER, Arnaud, F-63200 Riom (FR)
(74) Mandataire: Vaillant, Jeanne
(86) Numéro de dépôt international: FR9700913
(87) Numéro de publication internationale: WO97045101

(56) Documents cités:
- EP-A- 0 255 041
- EP-A- 0 334 498
- WO-A-93/04185
- WO-A-94/12259
- FR-A- 2 680 373
- FR-A- 2 730 931
- J. AM. OIL CHEM. SOC., vol. 67, no. 12, Décembre 1990, ILLINOIS, pages 996-1001, XP000644758 SHUICHI MATSUMURA ET AL.: "SURFACE ACTIVITIES, BIODEGRADABILITY AND ANTIMICROBIAL PROPERTIES OF n-ALKYLGLUCOSIDES, MANNOSIDES AND GALACTOSIDES."

## Description

La présente invention concerne l'utilisation d' α-alkylglucosides et/ou d'esters d'α-alkylglucoside, plus particulièrement d'esters d'α-butylglucoside, en tant qu'agent anti-microbien, notamment anti-bactérien et/ou anti-fongique, pour la préparation de compositions pharmaceutiques, cosmétiques ou agro-alimentaires, ou autres types de compositions dès l'instant où la présence d'un tel agent anti-microbien s'avère nécessaire. L'invention se rapporte en outre à des compositions spécifiques de telles utilisations et comprenant de tels agents anti-microbiens.

La protection des produits cosmétiques, dermatologiques, pharmaceutiques et agro-alimentaires contre un éventuel développement microbien nécessite l'emploi de conservateurs. Cependant, la plupart des conservateurs actuellement utilisés, notamment en cosmétique et dans les spécialités pharmaceutiques topiques, sont susceptibles de provoquer des irritations cutanées et occulaires. En outre, des incompatibilltés avec les composants utilisés, notamment certains tensioactifs, sont possibles.

Les esters d'acide gras de sucre ou d'alkylglucoside sont connus pour leurs propriétés émulsionnantes et conditionnantes vis à vis des cheveux et de la peau. Leurs utilisations en cosmétique sont décrites dans de nombreux articles et demandes de brevets.

De plus, le document Shuichi Matsumura et al., JAOCS, Vol. 67, n° 12 p. 996-1001 (Dec 1990), et décrit des propriétés antimicrobiennes des α-alkylglucosides dont le groupe alkyle comporte 8, 10 ou 12 atomes de carbone.

De façon inattendue, les inventeurs ont découvert que les esters d'α-alkylglucoside possèdent, outre les propriétés émulsionnantes qu'on connait, des propriétés bactéricides et/ou fongicides. Parallèlement, il a été découvert que des α-alkylglucosides, dont certains pourraient constituer le substrat de la réaction d'estérification fournissant lesdits esters, possèdent aussi certaines propriétés antimicrobiennes, notamment bactéricides.

Un but de la présente invention est donc de fournir des moyens permettant d'empêcher ou de réduire efficacement des développements microbiens dans un milieu qui y est propice, tout en évitant les problèmes inhérents à l'utilisation de certains types de consetvateurs tel que précédemment évoqué.

La présente invention a donc pour principal objet l'utilisation dans une composition, notamment pharmaceutique, cosmétique ou agro-alimentaire, d'un composant α-alkylglucoside ou d'un composant ester d'α-alkylglucoside ou un mélange de ceux-ci, en tant qu'agent microbicide, notamment bactéricide ou fongicide ou les deux à la fois, ledit composant ou ledit mélange étant en proportion suffisante pour préserver ladite composition d'un développement microbien notamment bactérien ou fongique ou les deux à la fois.

Il est entendu que la présente invention couvre également l'utilisation de mélanges d'au moins deux composants différents esters d'α-alkyl glucoside et/ou α-alkyl glucosides.

Le pouvoir conservateur de tels composés permet donc un effet « auto-protecteur » contre la contamination microbienne des formules pharmaceutiques, cosmétiques, dermatologiques, ou agro-alimentaires. Les esters d'α-alkylglucosides, et notamment les esters d'α-butylglucoside, peuvent être obtenus par estérification enzymatique à partir d'α-alkylglucoside tel que cela est décrit dans le brevet d'invention PCT/FR92/00782. Les produits obtenus par ce procédé sont stéréospécifiques (α) et sont normalement des monoglucosides. En raison de l'absence d'anomère β dans les produits utilisés selon la présente invention, certaines caractéristiques physiques, telles que le point de fusion et la solubilité de l'alkylglucoside sont définies de façon très précise.

Les α-alkylglucosides sont estérifiés de préférence dans les positions C₆ puis C₂. Cette estérification enzymatique est catalysée par un préparation enzymatique ayant une activité de lipase. Les conditions de réaction permettent soit d'enrichir la proportion en monester d'α-alkytglucosides, estérification majoritairement sur le C₆, soit d'enrichir la proportion en diester, estérification majoritairement en C₂ et C₆. Le procédé mis en oeuvre permet d'estérifier aussi bien les acides gras saturés que les acides gras insaturés.

De tels esters sont stéréospécifiques, chimiquement purs et parfaitement caractérisés. Ils ne se retrouvent en outre pas mêlés à des produits secondaires, dés lors qu'ils sont obtenus par un procédé de synthèse entièrement enzymatique. Ces esters d'α-alkylglucosides étant en outre des agents émulsifiants non toxiques et non irritants, sont ainsi particulièrement appropriés à une utilisation cosmétique, pharmaceutique ou alimentaire.

Dans une utilisation préférée selon la présente invention, le composant ester d'α-alkylglucoside, α-alkylglucoside ou leur mélange est en proportion comprise entre environ 0,5% et 10%, de préférence entre environ 2,5% et 5% en poids de la susdite composition.

En outre, le composant ester d'α-alkylglucoside est, en tant qu'agent bactéricide, en proportion au moins égale à environ 3,5 %, de préférence 5%, en poids de la composition.

Dans une utilisation préférée, le groupe alkyle de l'α-alkylglucoside ou de l'ester d'α-alkylglucoside est un groupe comportant 1 à 6 atomes de carbone, de préférence un groupe butyle.

De préférence, dans une telle utilisation l'ester d'α-butylglucoside représente au moins 40%, de préférence 80%, en poids de l'agent bactéricide et/ou fongicide.

Dans une utilisation particulièrement préférée, l'ester d'α-alkylglucoside est un mono- ou di- caprate d'α-butylglucoside, un mono- ou di- palmitate d'α-butylglucoside, un mono- ou di- cocoate d'α-butylglucoside.

Parmi les esters pouvant également être utilisés on citera les laurates, les myristates et les stéarates.

Ainsi que décrit ci-dessus, les inventeurs ont également découvert les propriétés bactéricides et fongicides d'α-alkylglucosides,. Dans une utilisation préférée de la présente invention, le composant α-alkylglucoside est, en tant qu'agent bactéricide, en proportion comprise entre environ 0,8% et 5% poids de la composition et, en tant qu'agent fongicide, en proportion comprise entre environ 1% et 3% en poids de la composition.

Selon les conditions dans lesquelles s'effectue l'utilisation selon la présente invention, il peut arriver que, dans une certaine mesure, l'activité microbicide des susdits composants varie. Il est entendu qu'il est dès lors tout à fait à la portée de l'homme du métier d'adapter ces conditions pour l'obtention de l'effet microbicide recherché.

La présente invention se rapporte également à une composition pharmaceutique, cosmétique ou agro-alimentaire dont le principe actif comprend un ester d'α-alkylglucoside, un α-alkylglucoside ou un mélange ce ceux-ci, dont le groupe alkyle comporte 1 à 6 atomes de carbone. De manière préférée, ce principe actif est présent à raison de 0,5% à 10%, de préférence 2,5% à 5%, en poids par rapport à ladite composition.

De préférence, le groupe alkyle de l'α-alkylglucoside et/ou de l'ester d'α-alkylglucoside est un groupe comportant 1 à 6 atomes de carbone, de préférence un groupe butyle.

Dans une composition préférée de l'invention, l'ester d'α-alkylglucoside est un mono- ou di- caprate d'α-butylglucoside, un mono- ou di- palmitate d'α-butylglucoside, un mono- ou di- cocoate d'α-butylglucoside.

Dans une composition particulièrement préférée, le principe actif est constitué de l'un des mélanges a), b), c), et d) suivants :

| | | |
|---|---|---|
| a) | Polyéthylène glycol (30) dipolyhydroxystéarate | 15 % |
| | Monocaprate d'α-butylglucoside | 48 % |
| | Dipalmitate d'α-butylglucoside | 37 % |
| b) | Ester de polyoxyde d'éthylène et d'alcool gras | 40 % |
| | Ether de polyethylène glycol (21) et alcool stéarique | 15 % |
| | Monocaprate d'α-butylglucoside | 26 % |
| | Monopalmitate d'α-butylglucoside | 19 % |
| c) | Ester d'acide citrique et glyceryl sorbitol | 20 % |
| | Monocaprate d'α-butylglucoside | 46 % |
| | Monopalmitate d'α-butylglucoside | 34 % |
| d) | Dicocoate d'α-butylglucoside | 48 % |
| | Monococoate d'α-butylglucoside | 37 % |
| | Monopalmitate d'α-butylglucoside | 15 % |

Ces mélanges a), b), c),et d) ont été respectivement dénommés : BEC 4, BEC 721985, BEC SCS et BEC SH.

De préférence encore, le pH d'une composition selon l'invention, est compris entre 3 et 10, et de préférence environ égal à 5.

La présente invention se rapporte en outre à un procédé de préparation d'un ester d'α-butylglucoside pour une utilisation selon l'invention, dans lequel ledit ester d'α-butylglucoside est obtenu par estérification enzymatique stéréospécifique d'α-butylglucoside en présence d'un agent déviscosifiant, notamment l'hexane

La présente invention concerne également un procédé de traitement anti-microbien externe, notamment chez l'humain, dans lequel les composés du type α-alkylglucosides et esters d'α-alkylglucosides précédemment décrits agissent en tant qu'agents anti-bactériens et/ou anti-fongiques, sous différentes formes galléniques.

Les inventeurs ont donc testé l'activité bactéricide et/ou fongicide de composés du type α-alkylglucosides et esters d'α-alkylglucosides afin de déterminer les conditions, notamment les concentrations, dans lesquelles lesdits composés doivent être utilisés pour que les activités recherchées soient obtenues. Les résultats de ces tests ont été analysés conformément à la Pharmacopée Française qui décrit une méthodologie de contrôle de l'efficacité des agents de conservation anti-microbiens dans les préparations pharmaceutiques et établit des critères d'efficacité minimale en fonction des voies d'administration du médicament. Ainsi, des critères spécifiques sont fixés pour les spécialités topiques que l'on peut considérer comme proches des produits cosmétiques dans leur conception. Les critères recommandés et significatifs de l'efficacité bactéricide et/ou fongicide d'un produit sont :
- pour un effet bactéricide (*Staphylococcus aureus*, *Pseudonmonas aeruginosa, Eschérichia Coli)*
- réduction de 3 unités logarithmiques en 14 jours par rapport à la population initiale,
- aucune augmentation de la croissance microbienne après 14 jours et jusqu'au 28 ème jour, date d'arrêt du test;
- pour un effet fongicide (levures et moisissures) *(Candida albicans, Aspergillus niger)*
- réduction d'1 unité logarithmique en 14 jours par rapport à la population initiale,
- aucune augmentation de la croissance microbienne après 14 jours et jusqu'au 28 ème jour, date d'arrêt du test.

### EXPRESSION DES RESULTATS

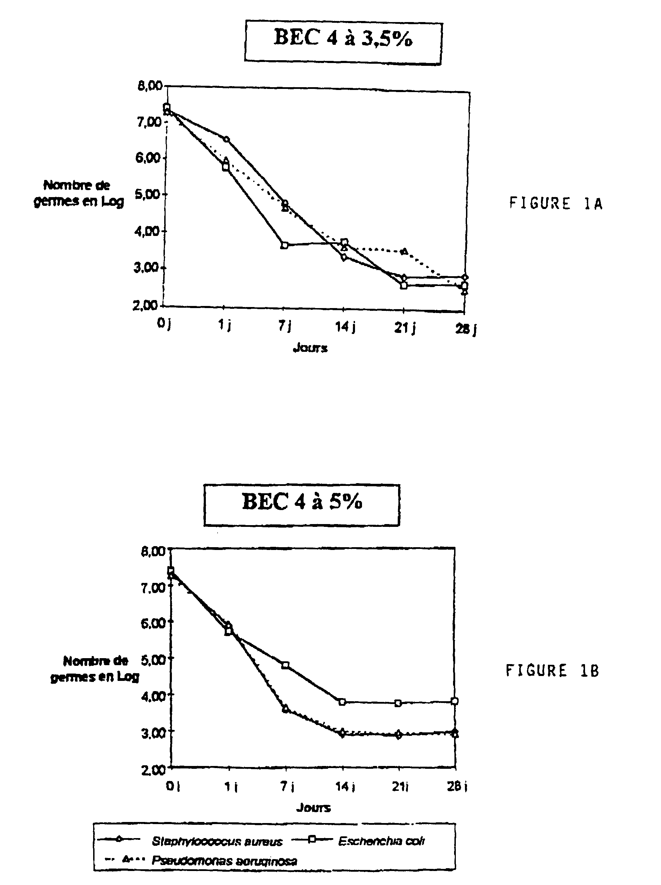

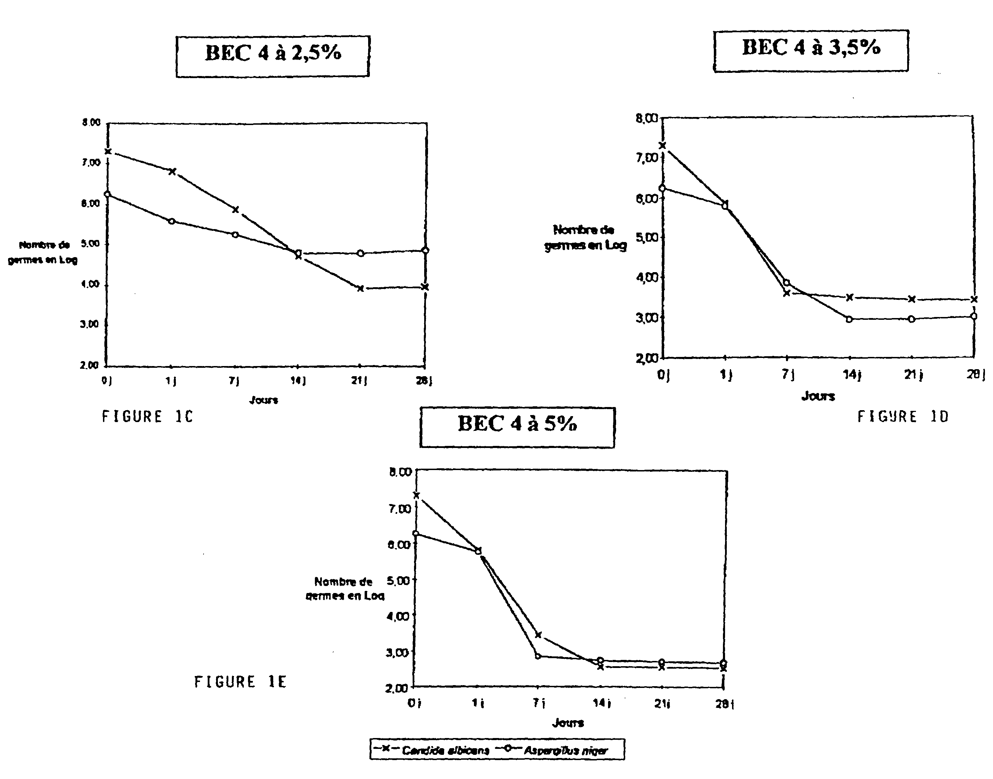

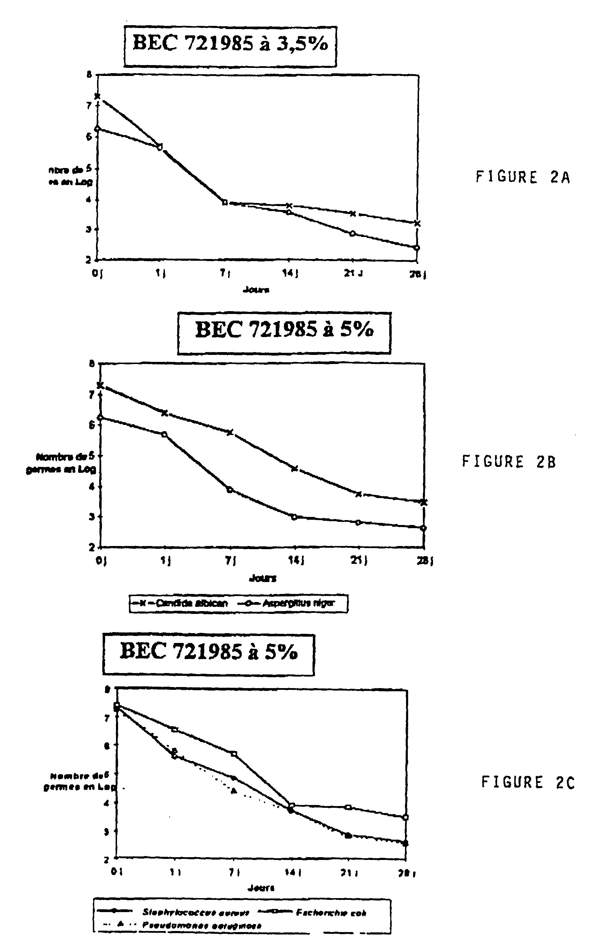

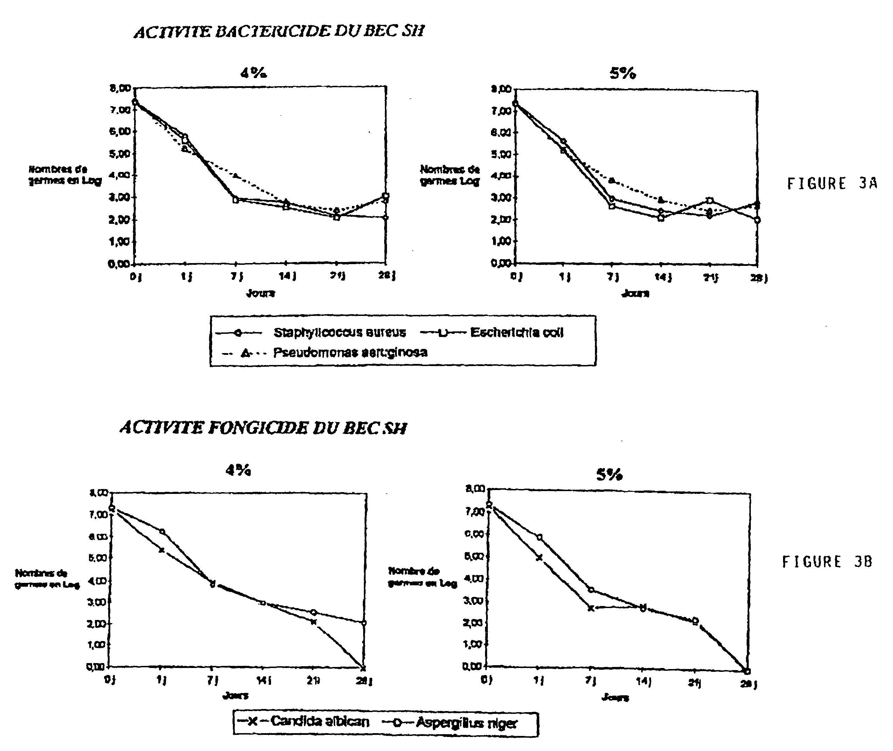

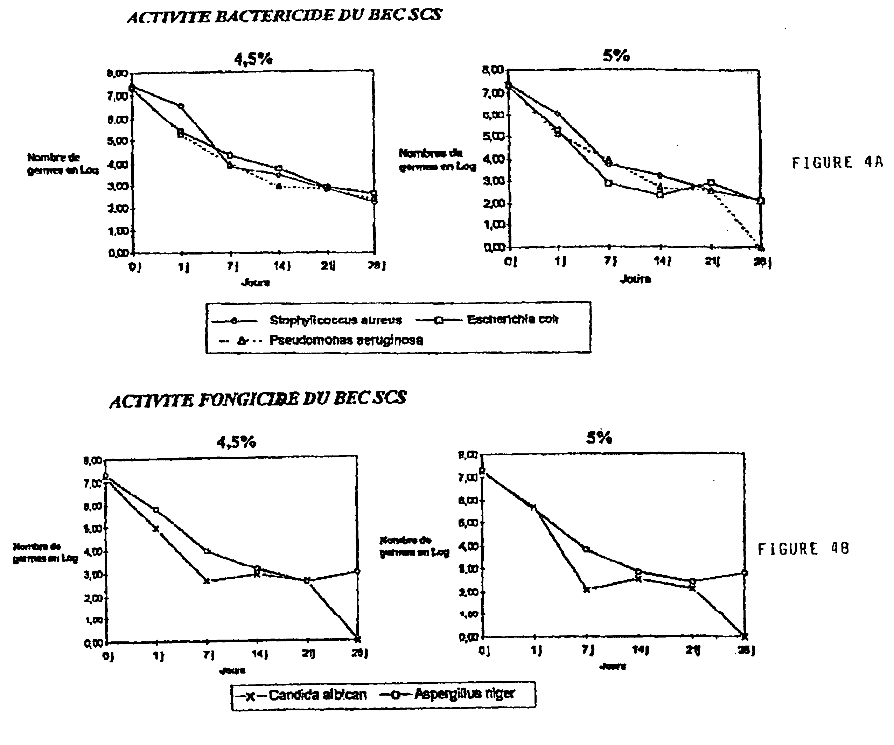

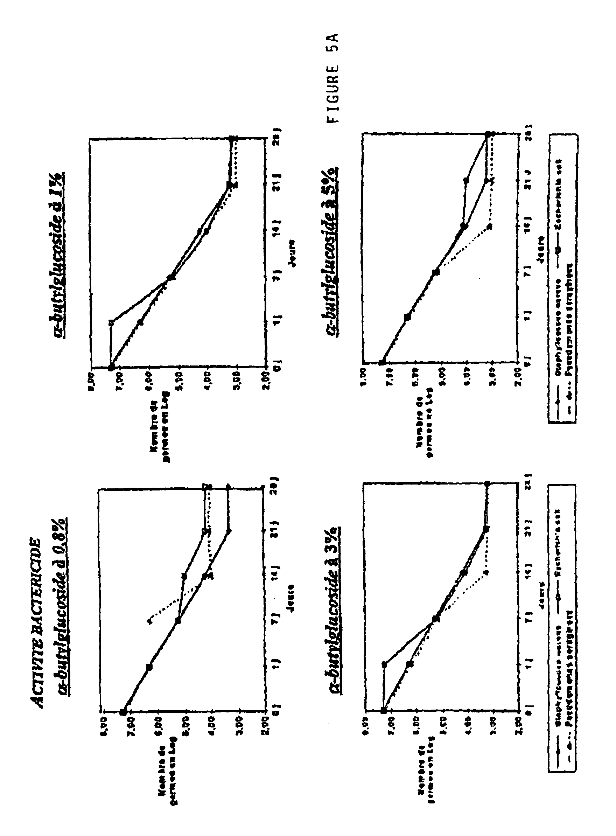

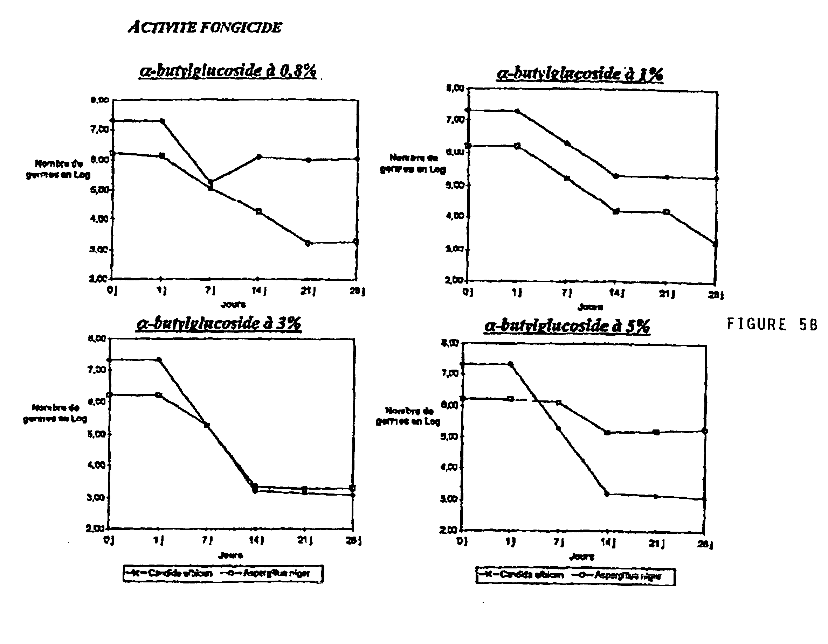

Afin de faciliter la visualisation de l'effet bactéricide ou fongicide des composés testés, les résultats obtenus ont été transcrits dans les figures 1 à 5 sous la forme de graphes présentant la diminution du nombre de germes en unité logarithmique, au cours du temps ( 1 jour, 7 jours, 14 jours, 21 jours, 28 jours).

### Bref descriptif des figures

La figure 1 montre l'activité anti-microbienne du mélange BEC 4 par représentation de la diminution du nombre de germes (Log ) bactériens ( Fig 1A et 1B) et fongiques (Fig 1C, 1D, 1E) à différentes concentrations, en fonction du temps (jours) chez différentes souches .

La figure 2 montre l'activité anti-microbienne du mélange BEC 721985 par représentation de la diminution du nombre de germes (Log ) bactériens (Fig 2C) et fongiques (Fig 2A et 2B), à différentes concentrations, en fonction du temps (jours) chez différentes souches.

La figure 3 montre l'activité anti-microbienne du mélange BEC SCS par représentation de la diminution du nombre de germes (Log ) bactériens(Fig3A) et fongiques (Fig3B), à différentes concentrations, en fonction du temps (jours) chez différentes souches.

La figure 4 montre l'activité anti-microbienne du mélange BEC SH par représentation de la diminution du nombre de germes (Log ) bactériens (Fig4A) et fongiques(Fig4B), à différentes concentrations, en fonction du temps (jours) chez différentes souches.

La figure 5 montre l'activité anti-microbienne d'α-alkylglucosides par représentation de la diminution du nombre de germes (Log ) bactériens (Fig5A) et fongiques (Fig5B), à différentes concentrations, en fonction du temps (jours) chez différentes souches .

Les résultats ont également été repris sous la forme de tableaux (voir ci-après) présentant les réductions logarithmes obtenues selon les différentes souches bactériennes ou fongiques selon lesquelles les tests ont été mis en oeuvre.

### ACTIVITE ANTIMICROBIENNE

Les tests effectués ont permi de démontrer l'activité anti-microbienne de certains types de composés ou de certains mélanges de composés. Il est entendu que les composés ou mélanges suivants, qui constituant les agents actifs, ne limitent aucunement la portée de l'utilisation selon la présente l'invention.

### A/ Activité antimicrobienne du monocaprate d'α-alkylglucosides

### Propriétés bactéricides et fongicides du monocaprate d'α-butylglucoside (α-BG) à 3 %, 3,5 %, 4 %, 4,5 % et 5 % ;

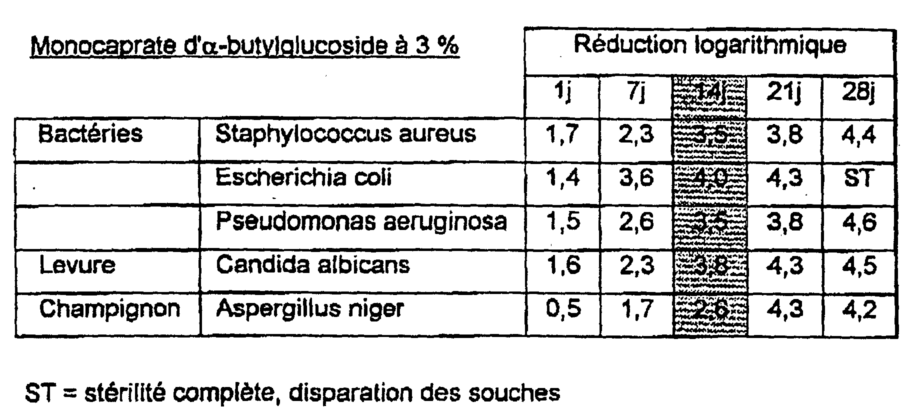

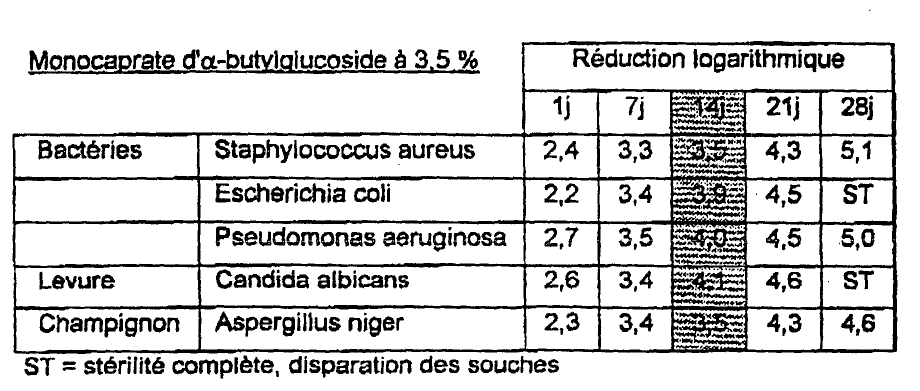

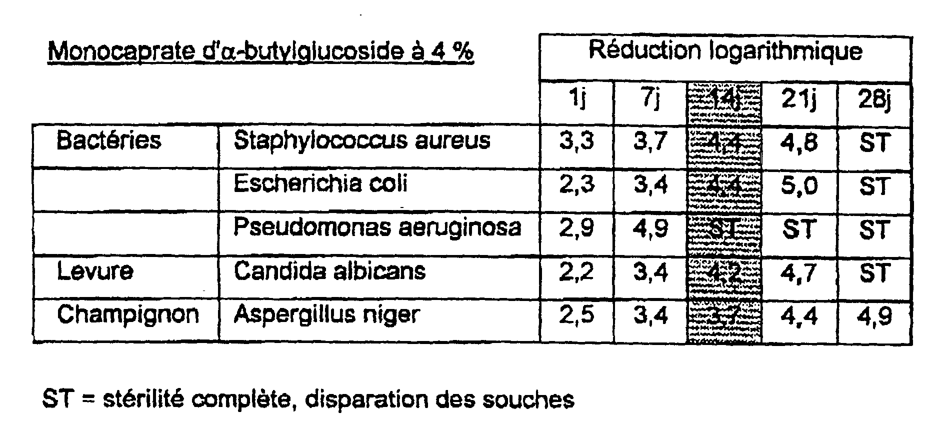

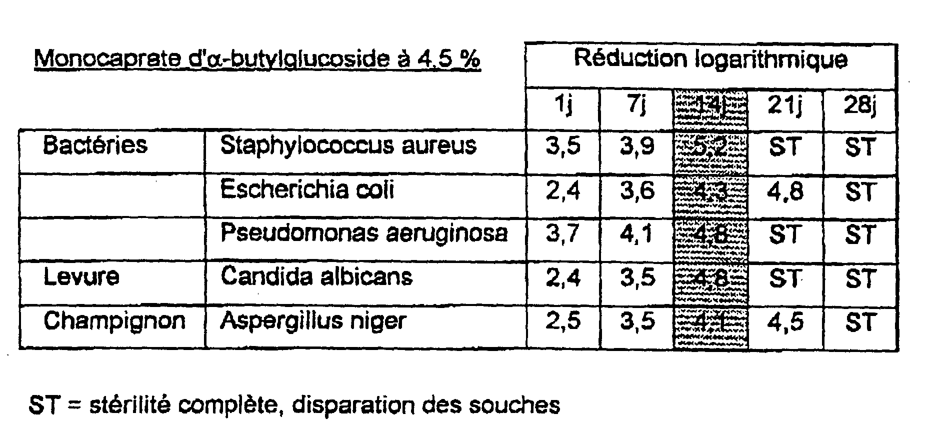

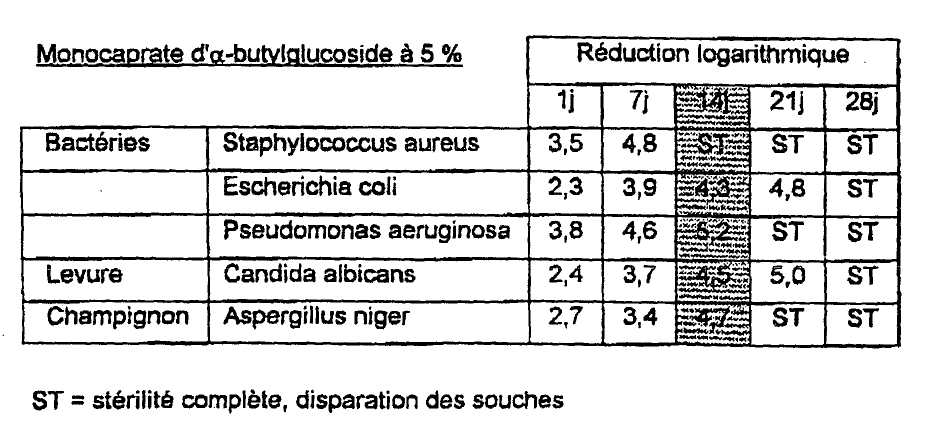

### B/ Activité antimicrobienne des esters d'α-alkylglucosides

Des esters d'α-alkylglucoside, plus particulièrement d'α-butylglucoside ont été associés ou non à d'autres composants pour former les mélanges ci-après dénommés « BEC »:

| | | |
|---|---|---|
| BEC 4 | Polyéthylène glycol (30) dipolyhydroxystéarate | 15 % |
| | Monocaprate d'α-butylglucoside | 48 % |
| | Dipalmitate d'α-butylglucosides | 37 % |
| | | |
| BEC 721 985 | Ester de polyoxyde d'éthylène et d'alcool gras | 40 % |
| | Ether de polyéthylène glycol (21) et alcool stéarique | 15 % |
| | Monocaprate d'α-butylglucoside | 26 % |
| | Monopalmitate d'α-butylglucoside | 19 % |
| | | |
| BEC SCS | Mélange d'ester d'acide citrique et glyceryl sorbitol | 20 % |
| | Monocaprate d'α-butylglucoside | 46 % |
| | Monopalmitate d'α-butylglucoside | 34 % |
| | | |
| BEC SH | Dicocoate d'α-butylglucoside | 48 % |
| | Monococoate d'α-butylglucoside | 37 % |
| | Monopalmitate d'α-butylglucoside | 15 % |

La proportion des mélanges ci-dessus dans les différentes compositions est exprimée dans les tableaux suivants en pourcentage en poids par rapport à ladite composition.

### I - Efficacité anti-microbienne du mélange BEC 4

### a) Activité bactéricide du mélange BEC 4 à 3,5% et 5% en poids par rapport à la composition totale.

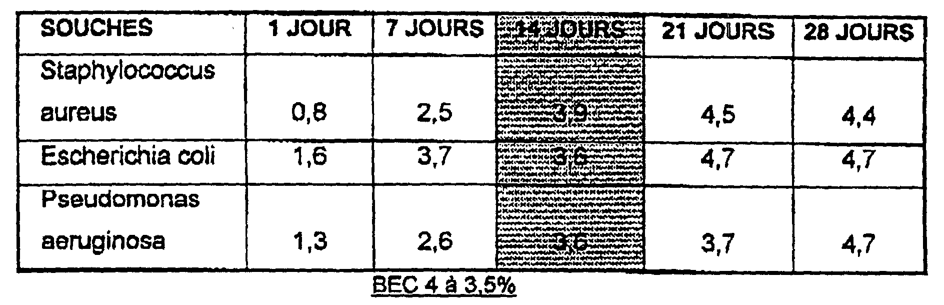

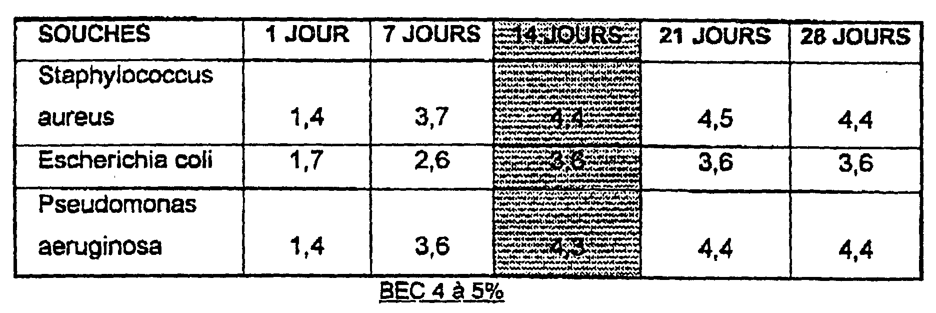

Conformément aux spécifications de la Pharmacopée Française, le mélange BEC 4 a induit un effet bactéricide important à 3,5 %, avec une réduction logarithmique de plus de 3 au bout de 14 jours. Cet effet s'accentue pour une concentration de 5 %, notamment pour les souches *Staphylococcus aureus* et de *Pseudomonas aeruginosa.*

### b) Activité fongicide du mélange BEC 4 à 2,5%, 3,5% et 5% en poids par rapport à la composition totale.

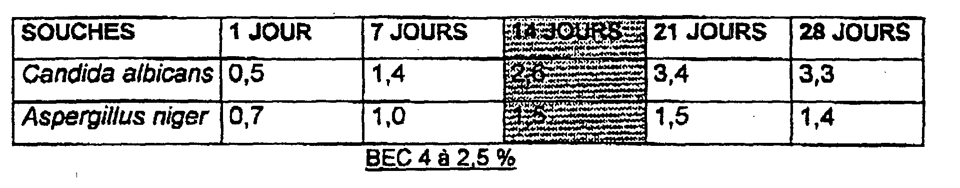

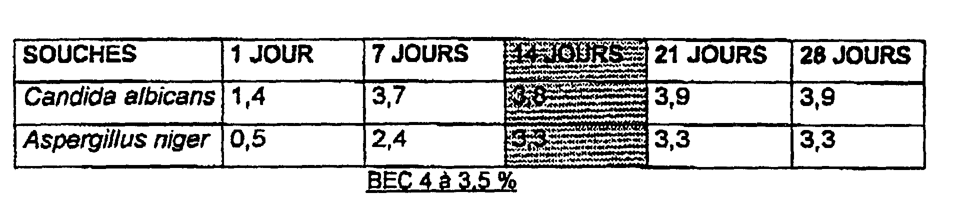

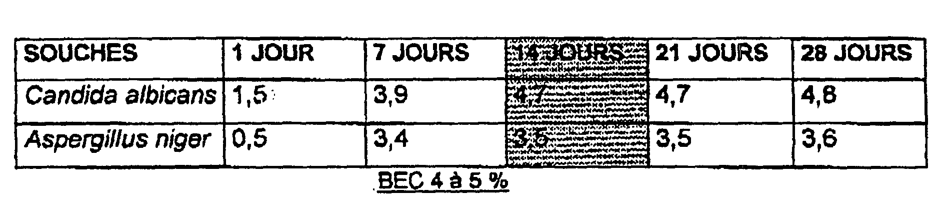

Conformément aux spécifications de la Pharmacopée Française le mélange BEC 4 induit un effet fongicide supérieur aux exigences minimales. Cet effet s'accentue notoirement en fonction des concentrations croissantes utilisées. Ainsi pour *Candida albicans*, les réductions logarithmiques sont de 2,6 / 3,8 / 4,7 pour des concentrations respectivement de 2,5 % / 3,5% / 5%. Pour *Aspergillus niger,* l'augmentation des réductions logarithmiques en fonction de la concentration est plus faible : elles passent de 1,5 / 3,3 / 3,5 pour des concentrations respectivement de 2,5 % / 3,5% / 5%.

### II - Efficacité anti-microbienne du mélange BEC 721985

### a) Activité bactéricide du mélange BEC 721985 à 5% en poids par rapport à la composition totale

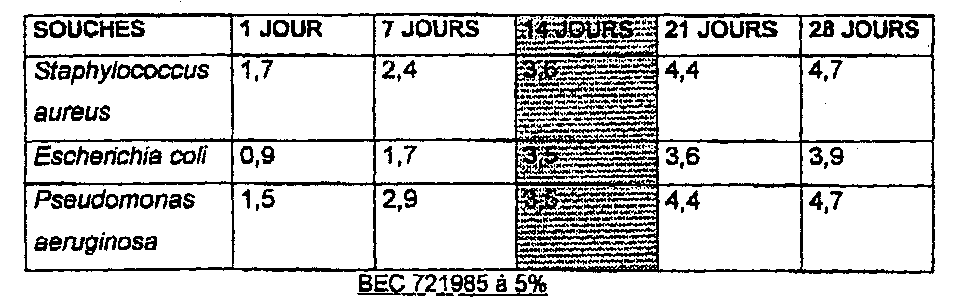

Le mélange BEC 721985 a induit un effet bactéricide important à partir d'une concentration de 5% , avec une réduction logarithmique supérieure à 3 au bout de 14 jours.

### b) Activité fongicide du mélange BEC 721985 à 3,5% et 5% en poids par rapport à la composition totale.

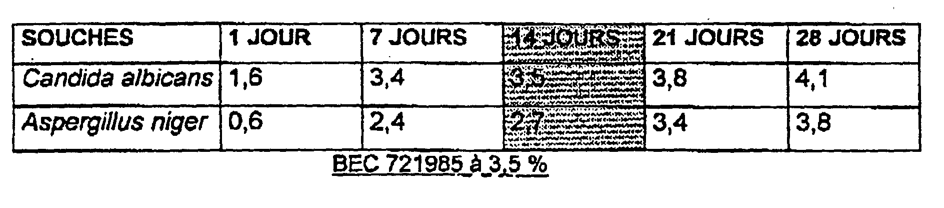

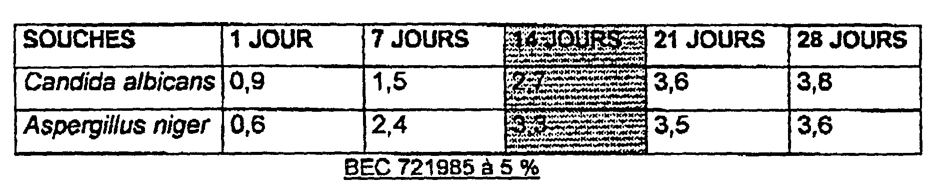

Le mélange BEC 721985 induit un effet fongicide important à partir de 3,5%. Ainsi pour *Candida albicans*, les réductions logarithmiques varient de 3,5 à 2,7 pour des concentrations respectivement de 3,5 et 5%. Pour *Aspergillus niger*, les réductions logarithmiques sont de 2,7 et 3,3 pour des concentrations respectivement de 3,5 et 5%.

### III - Efficacité anti-microbienne du mélange BEC SH

### a) Activité bactéricide de BEC SH à 4% et 5% en poids par rapport à la composition totale.

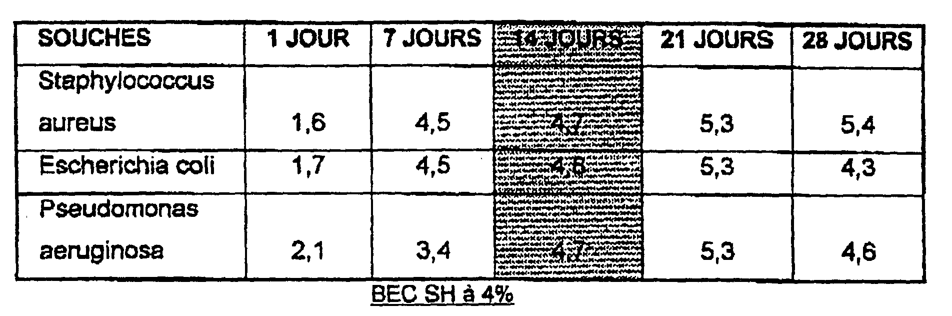

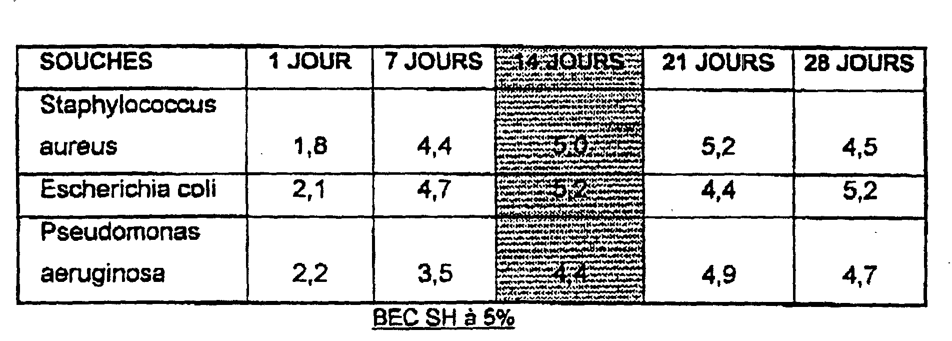

### b) Activité fongicide de BEC SH à 4% et 5% en poids par rapport à la composition totale.

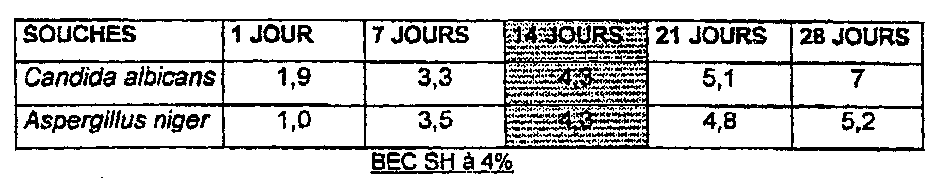

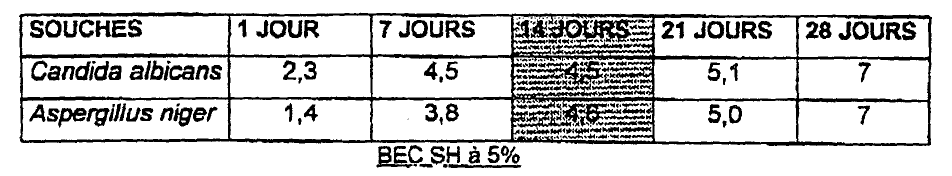

### IV - Efficacité anti-microbienne du mélange BEC SCS à 4,5% et 5% en poids par rapport à la composition totale.

### a) Activité bactéricide de BEC SCS

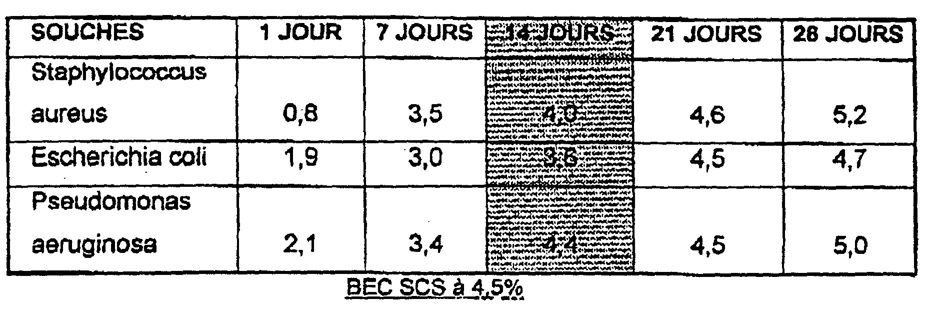

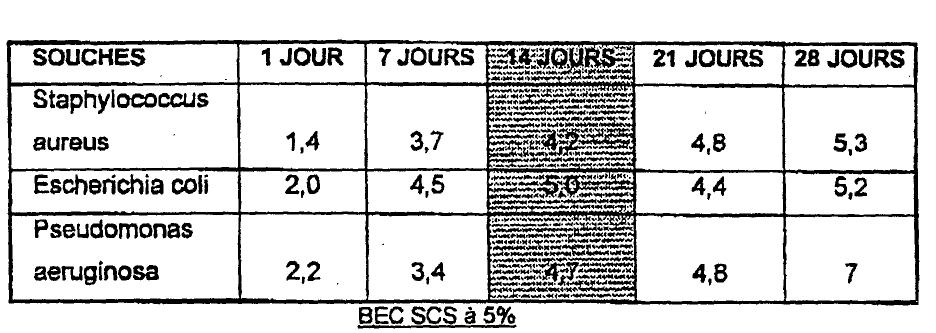

### b) Activité fongicide de BEC SCS à 4.5% et 5% en poids par rapport à la composition totale.

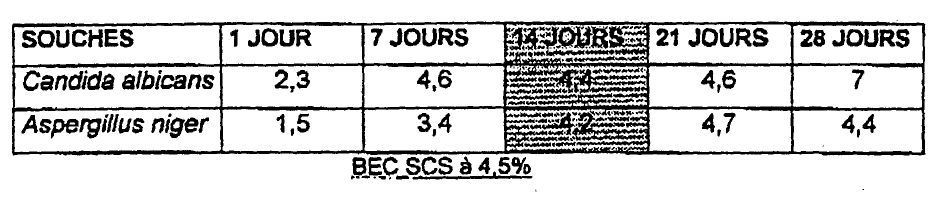

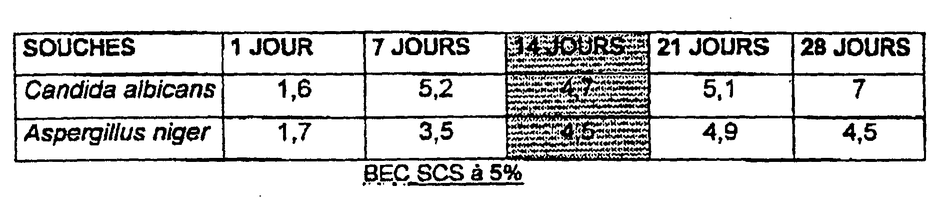

L'incorporation dans des formules cosmétiques des esters α-butylglucoside est résumée dans le tableau suivant :

| **α-Butyl** **glucoside** | **Formulation** | **Dose** **Recommandée** **pour effet** **émulsionnant** | **Activité** **Bactéricide** | **Activité** **Fongicide** |
|---|---|---|---|---|
| BEC 4 | Emulsion E/H stables, blanches, très onctueuses | 2.5-5% | à partir de 3,5% | à partir de 2,5% |
| BEC 721985 | Emulsion H/E stables, blanches, très onctueuses. | 2.5-5% | à partir de 5% | à partir de 3,5% |
| BEC SCS | Emulsion H/E stables, blanches, très onctueuses. | 2.5-5% | à partir de 3,5% | à partir de 3,5% |
| BEC SH | Produits d'hygiène doux, mousse onctueuse, blanche, satinée | 0.25 à 0.75% | à partir de 2,5% | à partir de 2,5% |

### C/ Activité antimicrobienne des d'α-butylglucosides

### Propriétés bactéricides et fongicides de l'α-butylglucoside (α-BG) à 0,8%, 1%, 3% et 5% en poids par rapport à la composition totale.

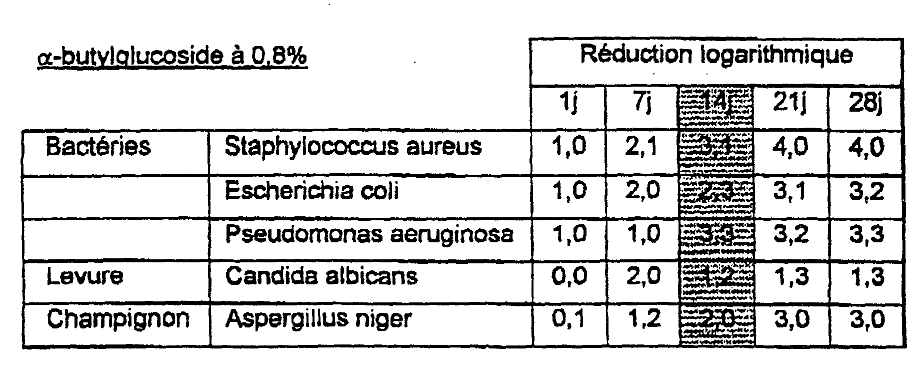

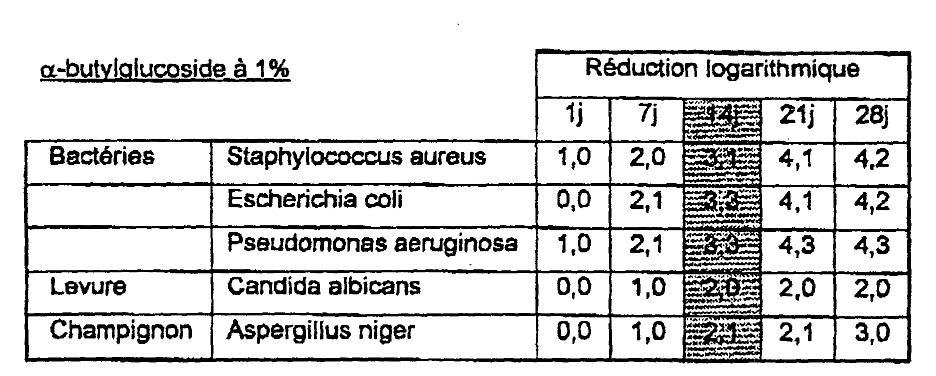

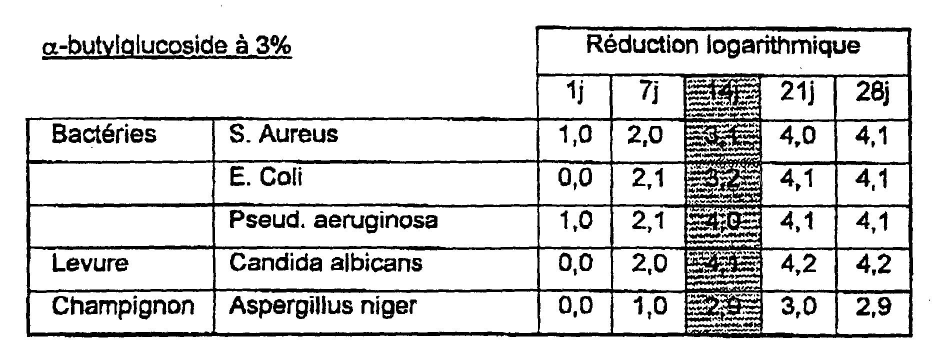

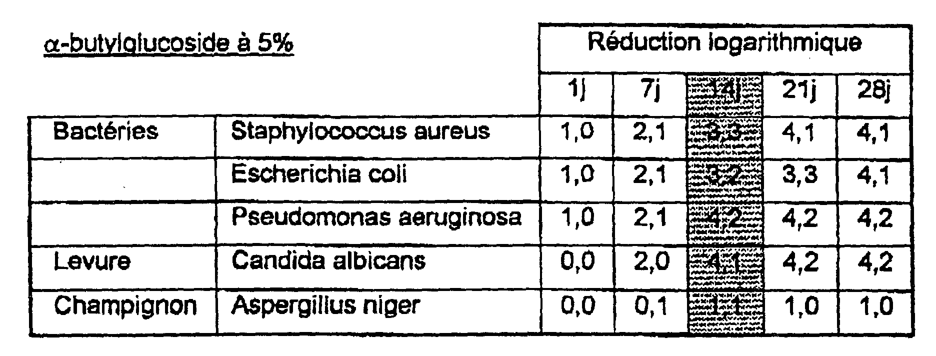

Les comptages ont traduit une faible diminution du nombre de germes pour des concentrations inférieures à 0,8%. L'α-BG correspond aux normes bactéricides exigées par la pharmacopée française pour des concentrations supérieures à 1%. On a observé une réduction logarithmique du nombre de souche de plus de 3 au bout de 14 jours sans augmentation ultérieure du nombre de souches. L'activité bactéricide de l'α-butylglucoside utilisé à des concentrations comprises entre 1 et 5% semble donc efficace. Il semblerait en outre qu'on obtienne un effet bactéricide dans un premier temps puis bactériostatique dans un second.

Les concentrations de 1 et 3% répondent aux normes fongicides. De façon surprenante, une concentration de 5% en αBG semblerait moins active sur *Aspergillus Niger* que des concentrations inférieures. Il y aurait donc un « effet dose » concernant le pouvoir fongicide de l'α-BG sur *Aspergillus Niger.*

### D/Applications particulières

Les propriétés bactéricides et fongicides permettent d'envisager l'utilisation des α-alkylglucosides, des esters d'α-alkylglucoside et des mélanges « BEC » en tant que principes actifs cosmétiques et pharmaceutiques notamment dans les utilisations suivantes :
- utilisation dans la composition de produits pour l'hygiène et/ou de traitement capillaire, notamment les shampooings antipelliculaires, de produits coiffants ou colorants.
- utilisation dans la composition de produits pour l'hygiène et/ou le traitement de la peau, concernant notamment l'acné, sous forme de crème, de lait, de gel ou de lotion parfumée, de produits de bain ou de douche, de produits de rasage ou de maquillage, de déodorants ou de perspirants.

Les produits de beauté, de soins, de toilette et capillaires conformes peuvent en outre contenir des ingrédients habituellement utilisés en cosmétique ou en dermatologie, tels que parfurns, colorants, autres conservateurs, agents séquestrants, des huiles végétales, animales ou synthétiques, des perfluoropolyethers, des agents hydratants, des agents antirides, des amincissants, des filtres solaires, des agents tensioactifs anioniques, non-ioniques, amphotères ou cationiques, des polymères, des protéines, des agents de conditionnement, des stabilisateurs de mousse, des propulseurs.

### E/ Exemples de compositions

Les tableaux ci-après présentent des compositions comprenant les principes actifs dans leur utilisation selon l'invention. Ces exemples sont destinés à illustrer la présente invention sans pour autant présenter un caractère limitatif.

### Exemple 1

| EMULSION E/H BEC 4/2,5% | | | | |
|---|---|---|---|---|
| PHASE | INGREDIENTS | NOM INCI | % | FOURNISSEUR |
| A | BEC 4 | | 2,5 | SOLABIA® |
| A | Lanette O | Cetostearylic alcohol | 2 | HENKEL® |
| A | Cetiol J 600 | Jojoba Oil | 3,5 | HENKEL® |
| A | Arlamol E | PPG 15 stearyl ether | 4 | ICI® |
| A | Arlamol 812 | Capric/caprylic oil | 5 | ICI® |
| A | DC 200/350 Cs | Cyclomethicone | 1 | DOW |
| A | Cipol C₁₆ | Stearylic alcohol | 2,5 | CORNING® |
| A | Aerosil 972 | Silica | 0,5 | HENKEL® |
| A | Titane dioxyde | Titane Dioxyde | 10 | DEGUSSA® |
| A | Propylène glycol | Propylène glycol | 4 | |
| | Fucogel 1000 | Biosaccharide Gum-1 | 5 | |
| | Eau | | Q.S.P. 100 | SOLABIA® |

### Exemple 2

| EMULSION H/E BEC721985/5% | | | | |
|---|---|---|---|---|
| PHASE | INGREDIENTS | NOM INCI | % | FOURNISSEUR |
| A | BEC 721985 | | 5 | SOLABIA® |
| A | Lanette O | Cetostearylic alcohol | 2 | HENKEL® |
| A | Cetiol J 600 | Jojoba Oil | 3,5 | HENKEL® |
| A | Ariamol E | PPG 15 stearyl ether | 4 | ICI® |
| A | Ariamol 812 | Capric/caprylic oil | 5 | ICI® |
| A | DC 200/350 Cs | Cyclomethicone | 1 | DOW |
| A | Cipol C₁₆ | Stearylic alcohol | 2,5 | CORNING® |
| A | Aerosil 972 | Silica | 0,5 | HENKEL® |
| B | Propylène glycol | Propylène glycol | 4 | DEGUSSA® |
| B | Rhodicare T | Canthan Gum | 0,15 | RHONE P. @ |
| B | Eau | | Q.S.P. 100 | |

### Exemple3

| FORMULE BEC SH/0,75% | | | | |
|---|---|---|---|---|
| PHASE | INGREDIENTS | NOM INCI | % | FOURNISSEUR |
| G | BEC SH | | 0,75 | SOLABIA® |
| C | Miracare 2M CASE | Disodium cocoamphodiacetate (and) Sodium lauryl sulfate (and) sodium laureth sulfate (and) Propylène glycol | 30 | RHONE P. ® |
| B | EDTA | EDTA | 0,10 | |
| D | Dehyton K | Cocoamidopropyl betaine | 5 | HENKEL® |
| E | Plantarene PS 10 | Sodium Laureth sulfate (and) lauryl glucoside | 5 | HENKEL® |
| F | Comperian LS | Cocoamide DEA (and) Laureth 12 | 1 | HENKEL® |
| I | Citric Acid | Citric Acid | 0,4 | |
| H | Germaben II | Propylène glycol (and) Diazolidinyl Urea (and) Methylparaben (and) Propylparaben | 0,45 | ISP® |
| A | Eau | | Q.S.P . 100 | |

## Revendications

1. Utilisation dans une composition, notamment pharmaceutique, cosmétique ou agro-alimentaire, d'un composant α-alkylglucoside ou d'un composant ester d'α-alkylglucoside, dont le groupe alkyle de l'α-alkylglucoside ou de l'ester d'α-alkylglucoside comporte 1 à 6 atomes de carbone, ou un mélange de ceux-ci, en tant qu'agent anti-microbien ou fongicide ou les deux à la fois, ledit composant ou ledit mélange étant en proportion suffisante pour préserver ladite composition d'un développement microbien notamment bactérien ou fongique ou les deux à la fois.

2. Utilisation selon la revendication 1 dans laquelle ledit composant α-alkylglucoside ou ester d'α-glucoside, ou mélange de ceux-ci est utilisé, en tant qu'agent anti-bactérien ou fongicide ou les deux à la fois, en proportion suffisante pour préserver ladite composition d'un développement bactérien
ou fongique ou les deux à la fois.

3. Utilisation selon la revendication 1 ou 2, **caractérisée en ce que** le composant ou le mélange est en proportion comprise entre environ 0,5 % et 10 %, de préférence entre environ 2,5 % et 5 % en poids de ladite composition.

4. Utilisation selon la revendication 3, **caractérisée en ce que** le composant ester d'α-alkylglucoside est, en tant qu'agent bactéricide, en proportion au moins égale à environ 3,5 %, de préférence 5 %, en poids de la composition.

5. Utilisation selon l'une des revendications 1 à 4, **caractérisée en ce que** le groupe alkyle est un groupe butyle.

6. Utilisation selon l'une des revendications 1 à 5, **caractérisée en ce que** l'ester d'α-alkylglucoside représente au moins 40 %, de préférence au moins 80 %, en poids de l'agent bactéricide et/ou fongicide.

7. Utilisation selon l'une quelconque des revendications 1 à 6,
**caractérisée en ce que** l'ester d'α-alkylglucoside est un mono- ou di-caprate d'α-butylglucoside, un mono- ou di- palmitate d'α-butylglucoside, un mono- ou di-cocoate d'α-alkylglucoside.

8. Utilisation selon l'une quelconque des revendications 1 à 7 dans laquelle différents composants ester d'α-butylglucoside sont associés ou non à d'autres composants selon la répartition a), b), c) ou d) suivante :
| | | |
|---|---|---|
| a) | Polyéthylène glycol (30) dipolyhydroxystéarate | 15 % |
| | Monocaprate d'α-butylglucoside | 48 % |
| | Dipalmitate d'α-butylglucoside | 37 % |
| | | |
| b) | Ester de polyoxyde d'éthylène et d'alcool gras | 40 % |
| | Ether de polyéthylène glycol (21) et alcool stéarique | 15 % |
| | Monocaprate d'α-butylglucoside | 26 % |
| | Monopalmitate d'α-butylglucoside | 19 % |
| | | |
| c) | Ester d'acide citrique et glyceryl sorbitol | 20 % |
| | Monocaprate d'α-butylglucoside | 46 % |
| | Monopalmitate d'α-butylglucoside | 34 % |
| | | |
| d) | Dicocoate d'α-butylglucoside | 48 % |
| | Monocoate d'α-butylglucoside | 37 % |
| | Monopalmitate d'α-butylglucoside | 15 % |

9. Utilisation selon la revendication 1, 2 ou 3, **caractérisée en ce que** le composant α-alkylglucoside est, en tant qu'agent bactéricide, en proportion comprise entre 0,8 % et 5 % poids de la composition, et, en tant qu'agent fongicide, en proportion comprise entre environ 1 % et 3 % en poids de la composition.

10. Utilisation selon l'une quelconque des revendications 1 à 9 dans la composition de produits pour l'hygiène et/ou de traitement capillaire, notamment les shampooings antipelliculaires, de produits coiffants ou colorants.

11. Utilisation selon l'une quelconque des revendications 1 à 9 dans la composition de produits pour l'hygiène et/ou le traitement de la peau, concernant notamment l'acné, sous forme de crème, de lait, de gel ou de lotion parfumée, de produit de bain ou de douche, de produits de rasage ou de maquillage, de déodorants ou de perspirants.

12. Composition pharmaceutique, cosmétique ou agro-alimentaire dont le principe actif comprend un ester d'α-alkylglucoside, un α-alkylglucoside ou un mélange de ceux-ci, dont le groupe alkyle comporte 1 à 6 atomes de carbone, **caractérisée en ce que** le principe actif est présent à raison de 0,5 à 10% en poids par rapport à ladite composition, et **en ce que** le principe actif est constitué de l'un des mélanges a), b), c) et d) suivants :
| | | |
|---|---|---|
| a) | Polyéthylène glycol (30) dipolyhydroxystéarate | 15 % |
| | Monocaprate d'α-butylglucoside | 48 % |
| | Dipalmitate d'α-butylglucoside | 37 % |
| | | |
| b) | Ester de polyoxyde d'éthylène et d'alcool gras | 40 % |
| | Ether de polyéthylène glycol (21) et alcool stéarique | 15 % |
| | Monocaprate d'α-butylglucoside | 26 % |
| | Monopalmitate d'α-butylglucoside | 19 % |
| | | |
| c) | Ester d'acide citrique et glyceryl sorbitol | 20 % |
| | Monocaprate d'α-butylglucoside | 46 % |
| | Monopalmitate d'α-butylglucoside | 34 % |
| | | |
| d) | Dicocoate d'α-butylglucoside | 48 % |
| | Monococoate d'α-butylglucoside | 37 % |
| | Monopalmitate d'α-butylglucoside | 15 % |

13. Composition selon la revendication 12, **caractérisée en ce que** le principe actif est présent à raison de 2,5% à 5% en poids par rapport à ladite composition.

14. Composition selon la revendication 12 ou 13, **caractérisée en ce que** son pH est compris entre 3 et 10.

15. Composition selon l'une des revendications 12 à 14, **caractérisée en ce que** son pH est environ égal à 5.

## Patentansprüche

1. Verwendung einer α-Alkylglucosid-Komponente oder einer α-Alkylglucosidester-Komponente, worin die Alkylgruppe des α-Alkylglucosids oder des α-Alkylglucosidesters 1 bis 6 Kohlenstoffatome umfasst, oder einer Mischung von diesen beiden als antimikrobielles oder fungizides Mittel oder als beides zugleich in einer Zusammensetzung, insbesondere pharmazeutischen, kosmetischen oder agrarwirtschaftlichen Zusammensetzung, wobei die Komponente oder die Mischung in einem ausreichenden Anteil vorhanden sind, um die Zusammensetzung vor einer mikrobiellen, insbesondere bakteriellen oder pilzlichen Vermehrung oder vor beidem zugleich zu schützen.

2. Verwendung nach Anspruch 1, in welcher die α-Alkylglucosid oder α-Alkylglucosidester-Komponente oder die Mischung von diesen als antibakterielles oder fungizides Mittel oder als beides zugleich in einem ausreichenden Anteil, um die Zusammensetzung vor einer bakteriellen oder pilzlichen Vermehrung oder vor beidem zugleich zu schützen, eingesetzt wird.

3. Verwendung nach Anspruch 1 oder 2, **dadurch gekennzeichnet, dass** die Komponente oder die Mischung in einem Anteil zwischen ungefähr 0,5 Gew.-% und 10 Gew.-%, vorzugsweise zwischen ungefähr 2,5 Gew.-% und 5 Gew.-% der Zusammensetzung vorhanden ist.

4. Verwendung nach Anspruch 3, **dadurch gekennzeichnet, dass** die α-Alkylglucosidester-Komponente als bakterizides Mittel in einem Anteil von wenigstens gleich ungefähr 3,5 Gew.-%, vorzugsweise 5 Gew.-% der Zusammensetzung vorhanden ist.

5. Verwendung nach einem der Ansprüche 1 bis 4, **dadurch gekennzeichnet, dass** die Alkylgruppe eine Butylgruppe ist.

6. Verwendung nach einem der Ansprüche 1 bis 5, **dadurch gekennzeichnet, dass** der α-Alkylglucosidester wenigstens 40 Gew.-%, vorzugsweise wenigstens 80 Gew.-% des bakteriziden und/oder fungiziden Mittels ausmacht.

7. Verwendung nach einem der Ansprüche 1 bis 6, **dadurch gekennzeichnet, dass** der α-Alkylglucosidester ein α-Butylglucosidmono- oder -dicaprat, ein α-Butylglucosidmono- oder - dipalmitat, ein α-Alkylglucosidmono- oder -dicocoat ist.

8. Verwendung nach einem der Ansprüche 1 bis 7, bei der verschiedene α-Butylglucosidester-Komponenten gegebenenfalls mit anderen Komponenten gemäß der folgenden Verteilung a), b), c) oder d) kombiniert sind:
| | | |
|---|---|---|
| a) | Polyethylenglycol (30) dipolyhydroxystearat | 15% |
| | α-Butylglucosidmonocaprat | 48% |
| | α-Butylglucosiddipalmitat | 37% |
| | | |
| b) | Ethylenpolyoxid-fettalkoholester | 40% |
| | Polyethylenglycol(21)-stearylalkoholether | 15% |
| | α-Butylglucosidmonocaprat | 26% |
| | α-Butylglucosidmonopalmitat | 19% |
| | | |
| c) | Citronensäure-glycerylsorbitolester | 20% |
| | α-Butylglucosidmonocaprat | 46% |
| | α-Butylglucosidmonopalmitat | 34% |
| | | |
| d) | α-Butylglucosiddicocoat | 48% |
| | α-Butylglucosidmonocoat | 37% |
| | α-Butylglucosidmonopalmitat | 15% |

9. Verwendung nach Anspruch 1, 2 oder 3, **dadurch gekennzeichnet, dass** die α-Alkylglucosid-Komponente als bakterizides Mittel in einem Anteil zwischen 0,8 Gew.-% und 5 Gew.-% der Zusammensetzung und als fungizides Mittel in einem Anteil zwischen ungefähr 1 Gew.-% und 3 Gew.-% der Zusammensetzung vorhanden ist.

10. Verwendung nach einem der Ansprüche 1 bis 9 in der Zusammensetzung von Produkten für die Hygiene und/oder die Haarbehandlung, insbesondere den Antischuppenshampoos, den Frisieroder Färbeprodukten.

11. Verwendung nach einem der Ansprüche 1 bis 9 in der Zusammensetzung von Produkten für die Hygiene und/oder die Behandlung der Haut, betreffend insbesondere Akne, in Form von Creme, Milch, Gel oder parfümierter Lotion, einem Bade- oder Duschprodukt, Rasier- oder Schminkprodukten, Deodorants oder Perspirants.

12. Pharmazeutische, kosmetische oder agrarwirtschaftliche Zusammensetzung, worin der Wirkstoff einen α-Alkylglucosidester, ein α-Alkylglucosid oder eine Mischung von diesen umfasst, deren Alkylgruppe 1 bis 6 Kohlenstoffatome umfasst, **dadurch gekennzeichnet, dass** der Wirkstoff in einer Menge von 0,5 bis 10 Gew.-% bezogen auf die Zusammensetzung vorhanden ist und dass der Wirkstoff aus einer der folgenden Mischungen a), b), c) und d) gebildet wird:
| | | |
|---|---|---|
| a) | Polyethylenglycol (30)dipolyhydroxystearat | 15% |
| | α-Butylglucosidmonocaprat | 48% |
| | α-Butylglucosiddipalmitat | 37% |
| | | |
| b) | Ethylenpolyoxid-fettalkoholester | 40% |
| | Polyethylenglycol(21)-stearylalkoholether | 15% |
| | α-Butylglucosidmonocaprat | 26% |
| | | |
| | α-Butylglucosidmonopalmitat | 19% |
| | | |
| c) | Citronensäure-glycerylsorbitolester | 20% |
| | α-Butylglucosidmonocaprat | 46% |
| | α-Butylglucosidmonopalmitat | 34% |
| | | |
| d) | α-Butylglucosiddicocoat | 48% |
| | α-Butylglucosidmonococoat | 37% |
| | α-Butylglucosidmonopalmitat | 15% |

13. Zusammensetzung nach Anspruch 12, **dadurch gekennzeichnet, dass** der Wirkstoff in einer Menge von 2,5 Gew.-% bis 5 Gew.-% bezogen auf die Zusammensetzung vorhanden ist.

14. Zusammensetzung nach Anspruch 12 oder 13, **dadurch gekennzeichnet, dass** ihr pH zwischen 3 und 10 liegt.

15. Zusammensetzung nach einem der Ansprüche 12 bis 14, **dadurch gekennzeichnet, dass** ihr pH ungefähr gleich 5 ist.

## Claims

1. Use in a composition, in particular in a pharmaceutical, cosmetic or agro-alimentary composition, of an α-alkylglucoside component or an α-alkylglucoside ester component or a mixture thereof, wherein the alkyl group of the α-alkylglucoside or the α-alkylglucoside ester contains 1 to 6 carbon atoms, as an antimicrobial agent or fungicide or both at the same time, said component or said mixture being in a proportion sufficient to preserve said composition from microbial development, in particular bacterial or fungal development, or both at the same time.

2. Use according to claim 1 wherein said α-alkylglucoside component or α-alkylglucoside ester component or mixture thereof is used as anti-bacterial or fungicide or both at the same time, in a proportion sufficient to preserve said composition from bacterial or fungal development, or both at the same time.

3. Use according to claim 1 or 2, **characterized in that** the component or mixture is in a proportion in the range about 0.5% to 10%, preferably in the range about 2.5% to 5%, by weight of said composition.

4. Use according to claim 3, **characterized in that** when it acts as a bactericidal agent, the α-alkylglucoside ester component is in a proportion of at least about 3.5%, preferably 5%, by weight of the composition.

5. Use according to one of claims 1 to 4, **characterized in that** the alkyl group is a butyl group.

6. Use according to one of claims 1 to 5, **characterized in that** the α-alkylglucoside ester represents at least 40%, preferably at least 80% by weight, of bactericidal and/or fungicidal agent.

7. Use according to any one of claims 1 to 6, **characterized in that** the α-alkylglucoside ester is an α-butylglucoside mono- or di-caprate, an α-butylglucoside mono- or di-palmitate, or an α-butylglucoside mono- or di-cocoate.

8. Use according to any one of claims 1 to 7 in which different α-butylglucoside ester components are combined or not combined with other components in the following mixtures a), b), c) or d):
| | | |
|---|---|---|
| a) | Polyethylene glycol (30) dipolyhydroxystearate | 15% |
| | α-butylglucoside monocaprate | 48% |
| | α-butylglucoside dipalmitate | 37% |
| | | |
| b) | Ester of polyethylene oxide and a fatty alcohol | 40% |
| | Ether of polyethylene glycol (21) and stearic alcohol | 15% |
| | α-butylglucoside monocaprate | 26% |
| | α-butylglucoside monopalmitate | 19% |
| | | |
| c) | Ester of citric acid and glyceryl sorbitol | 20% |
| | α-butylglucoside monocaprate | 46% |
| | α-butylglucoside monopalmitate | 34% |
| | | |
| d) | α-butylglucoside dicocoate | 48% |
| | α-butylglucoside monococoate | 37% |
| | α-butylglucoside monopalmitate | 15% |

9. Use according to claim 1 or claim 2 or claim 3, **characterized in that** when it acts as a bactericidal agent, the α-alkylglucoside component is in proportion in the range about 0.8% to 5% by weight of the composition, and when it acts as a fungicidal agent, it is in a proportion in the range about 1% to 3% by weight of the composition.

10. Use according to any one of claims 1 to 9 in a product composition for hygiene and/or hair treatment, in particular for an antidandruff shampoo, hair care product or dye.

11. Use according to any one of claims 1 to 9 in a product composition for a hygiene and/or skin treatment, in particular for the treatment of acne, in the form of a cream, a milk, a gel or a perfumed lotion, as a bath or shower product, as a shaving or make-up product, or as a deodorant or perspirant.

12. A pharmaceutical, cosmetic or agro-alimentary composition the active principle of which comprises an α-alkylglucoside ester, an α-alkylglucoside or a mixture thereof, the alkyl group of which comprises 1 to 6 carbon atoms, **characterized in that** the active principle is present in an amount of 0.5% to 10%, by weight with respect to said composition, and **in that** the active principle is constituted by one of the following mixtures a), b), c) and d):
| | | |
|---|---|---|
| a) | Polyethylene glycol (30) dipolyhydroxystearate | 15% |
| | α-butylglucoside monocaprate | 48% |
| | α-butylglucoside dipalmitate | 37% |
| | | |
| b) | Ester of polyethylene oxide and a fatty alcohol | 40% |
| | Ether of polyethylene glycol (21) and stearic alcohol | 15% |
| | α-butylglucoside monocaprate | 26% |
| | α-butylglucoside monopalmitate | 19% |
| | | |
| c) | Ester of citric acid and glyceryl sorbitol | 20% |
| | α-butylglucoside monocaprate | 46% |
| | α-butylglucoside monopalmitate | 34% |
| | | |
| d) | α-butylglucoside dicocoate | 48% |
| | α-butylglucoside monococoate | 37% |
| | α-butylglucoside monopalmitate | 15% |

13. A composition according to claim 12, **characterized in that** the active principle is present in an amount of 2.5% to 5% by weight with respect to said composition.

14. A composition according to claim 12 or claim 13, **characterized in that** its pH is in the range 3 to 10.

15. A composition according to one of claims 12 to 14, **characterized in that** its pH is about 5.
